# EUROPEAN PATENT SPECIFICATION

(11) **EP 3 380 899 B1**
(45) Date of publication and mention of the grant of the patent: **04.11.2020**
(21) Application number: 16823104.1
(22) Date of filing: 13.12.2016
(51) Int. Cl.: G06F 21/14, G06F 21/76, G05B 19/05

(54) **PROGRAM RANDOMIZATION FOR CYBER-ATTACK RESILIENT CONTROL IN PROGRAMMABLE LOGIC CONTROLLERS**
PROGRAMMRANDOMISIERUNG ZUR CYBERANGRIFFROBUSTEN STEUERUNG IN EINER SPEICHERPROGRAMMIERBAREN STEUERUNG
RANDOMISATION DE PROGRAMME POUR COMMANDE RÉSILIENTE POUR RÉSISTER AUX CYBERATTAQUES DANS DES AUTOMATES PROGRAMMABLES INDUSTRIELS

(30) Priority: 11.01.2016 US 201662277014 P
(43) Date of publication of application: 03.10.2018
(73) Proprietor: Siemens Aktiengesellschaft, 80333 München (DE)
(72) Inventor: MARTINEZ CANEDO, Arquimedes, Plainsboro, New Jersey 08536 (US)
(74) Representative: Isarpatent
(86) International application number: PCT/US2016/066295
(87) International publication number: WO 2017/123367

(56) References cited:
- EP-A1- 2 169 547
- SCHWARTZ MOSES ET AL: "Emerging Techniques for Field Device Security", 1 November 2014 (2014-11-01), SECURITY & PRIVACY, IEEE, IEEE SERVICE CENTER, LOS ALAMITOS, CA, US, PAGE(S) 24 - 31, XP011570172, ISSN: 1540-7993 [retrieved on 2015-01-12] page 25 page 29 - page 30
- MICHAEL FRANZ: "E unibus pluram", NEW SECURITY PARADIGMS, ACM, 2 PENN PLAZA, SUITE 701 NEW YORK NY 10121-0701 USA, 21 September 2010 (2010-09-21), pages 7-16, XP058312861, DOI: 10.1145/1900546.1900550 ISBN: 978-1-4503-0415-3
- Dimitrios Hristu-Varsakelis ET AL: "Handbook of Networked and Embedded Control Systems" In: "Handbook of Networked and Embedded Control Systems", 1 January 2005 (2005-01-01), Birkhäuser Boston, Boston, MA, XP055363099, ISBN: 978-0-8176-4404-8 pages 259-278, page 268
- FORREST S ET AL: "Building diverse computer systems", OPERATING SYSTEMS, 1997., THE SIXTH WORKSHOP ON HOT TOPICS IN CAPE COD, MA, USA 5-6 MAY 1997, LOS ALAMITOS, CA, USA,IEEE COMPUT. SOC, US, 5 May 1997 (1997-05-05), pages 67-72, XP010226847, DOI: 10.1109/HOTOS.1997.595185 ISBN: 978-0-8186-7834-9
- Siemens Ag Siemens Ag: "Programming Guideline for S7-1200/1500", , 1 March 2014 (2014-03-01), XP055362727, Retrieved from the Internet: URL:https://www.industry.siemens.nl/automa tion/nl/nl/industriele-automatisering/indu strial-automation/simatic-controller/modul aire-controllers/simatic-s7-1500/Documents /81318674_Programming_guideline_DOKU_v12_e n.pdf [retrieved on 2017-04-06]
- Anonymous: "Address space layout randomization - Wikipedia", , 2 December 2015 (2015-12-02), XP055363110, Retrieved from the Internet: URL:https://en.wikipedia.org/w/index.php?t itle=Address_space_layout_randomization&ol did=693416768 [retrieved on 2017-04-07]
- ANDREA HÖLLER ET AL: "Patterns for automated software diversity to support security and reliability", PATTERN LANGUAGES OF PROGRAMS, ACM, 2 PENN PLAZA, SUITE 701 NEW YORK NY 10121-0701 USA, 8 July 2015 (2015-07-08), pages 1-13, XP058079545, DOI: 10.1145/2855321.2855360 ISBN: 978-1-4503-3847-9

## Description

### TECHNICAL FIELD

The present invention relates generally to randomizing programs for cyber-attack resilient control in programmable logic controllers. The disclosed technology may be applied to, for example, various automated production environments where industrial controllers such as programmable logic controllers (PLCs) and distributed control systems (DCS) are used.

### BACKGROUND

Conventional resilient programmable logic controller (PLC) architectures are designed to withstand random and isolated faults caused, for example, by component or power failure. The so called "high availability" PLCs offer hot standby features where in the case of a crash, another PLC can take over the control of the system immediately, within the same cycle. Over decades, these features were sufficient to cope with hardware failure and standard software faults.

Unfortunately, conventional resilient architectures do not offer enough redundancy to cover faults originated by cyber-attacks because all the PLC instances are executing identical binaries in identical memory organization structures. Thus, a fault caused by a cyber-attack is likely to affect all the PLC instances and cause catastrophic consequences. In their academic paper, Höller et al. disclose methods for increasing the security and/or fault tolerance of software systems based on introducing "diversity in execution". This means that different versions of the same software show different characteristics during the execution while retaining the same functionality, (please refer to Höller A., Rauter T., Iber J., and Kreiner C., 2015. Patterns for Automated Software Diversity to Support Security and Reliability, in 2, 3, Article 1 (May 2015), 12 pages.)

### SUMMARY

Embodiments of the present invention address and overcome one or more of the above shortcomings and drawbacks, by providing methods, systems, and apparatuses related to randomizing programs for cyber-attack resilient control in PLCs. More specifically, binaries of PLC software are randomized in each of the instances of a redundant PLC architecture. Because the PLC instances are executing different binaries, the overall system has more resilience than conventional systems with respect to cyberattacks. Moreover, in some embodiments, each the memory organization structures of each PLC are also varied to provide additional protection from cyberattacks.

According to some embodiments, a method for PLC program randomization according to claim 1 is provided. It includes an engineering system computer receiving source code corresponding to a PLC program and compiling the source code into a plurality of functionally equivalent intermediate representations of the PLC program. The program structure of the PLC program is randomized during compilation such that each intermediate representation is unique among the plurality of intermediate representations. In some embodiments, randomization is performed prior to runtime of the program, while in other embodiments it may be performed at runtime, for example, using just-in-time compilation techniques generally known in the art. The engineering system computer transmits the plurality of intermediate representations to one or more PLCs.

In some embodiments of the aforementioned method for PLC program randomization, the PLC receives a first intermediate representation of the PLC program and compiles it into PLC assembly code. Program structure of the first intermediate representation is randomized during compilation. This PLC assembly code is then executed by the PLC. Following failover of the PLC, the first intermediate representation may be re-compiled into new PLC assembly code which is then executed by the PLC. The program structure of the first intermediate representation can be randomized again during re-compilation.

Various techniques may be applied to randomize program structure in the aforementioned method for PLC program randomization. For example, in some embodiments, the program structure of the PLC program is randomized during compilation by randomizing a memory layout of a plurality of data blocks used by the PLC program. For example, the memory layout of plurality of data blocks is randomized by assigning a unique memory address to each data block. The data blocks may be sorted by type during compilation to optimize memory access in the memory layout. In other embodiments, the program structure of the PLC program is randomized during compilation by randomizing usage of function blocks used by the PLC program. For example, the ordering of parameters used in calling each function block may be randomized or ordering cyclic variables used by each function block may be randomized. Additionally, usage of the plurality of function blocks may be randomized by constructing a control flow graph where conditional statements are transformed into equivalent control flow constructs or constructing a data flow graph where conditional statements are transformed into equivalent data flow expressions. Then, the order of the control flow constructs or data flow expressions may randomized. In other embodiments, function blocks usage is randomized inserting one or more non-functional function blocks into the plurality of function blocks. In one embodiment, each function block is identified by a triple comprising a numerical identifier and the program structure of the PLC program is randomized by assigning a random value to each numerical identifier and sorting the plurality of function blocks by numerical identifier. In some embodiments, the program structure of the PLC program is randomized during compilation by reordering one or more organization blocks in the PLC program.

According to other embodiments of the present invention, a method for PLC program randomization includes a PLC receiving an intermediate representation of a PLC program and compiling the intermediate representation into PLC assembly code. The program structure of the intermediate representation is randomized during compilation with respect to usage of one or more program blocks. The PLC can then execute the PLC assembly code. Following failover of the PLC, the PLC may re-compile the intermediate representation into a new PLC assembly code, again randomizing program structure of the intermediate representation during re-compilation. The new PLC assembly code may then be executed by the PLC.

In some embodiments of the aforementioned method for PLC program randomization, program structure of the intermediate representation is randomized during compilation by randomizing address values for one or more data blocks used by the PLC program. Other techniques may alternatively (or additionally) be used for randomizing the intermediate representation during compilation including (a) randomizing control flow of function blocks and functions used by the PLC program; (b) randomizing data flow of function blocks and functions used by the PLC program; and/or (c) reordering one or more organization blocks used by the PLC program in the intermediate representation.

According to other embodiments, a system for programmable logic controller (PLC) program randomization according to claim 13 is provided. It includes a computer readable storage medium storing source code corresponding to a PLC program; and engineering system and a plurality of programmable logic controllers. The engineering system is configured to compile the source code into a plurality of functionally equivalent intermediate representations of the PLC program. The program structure of the PLC program is randomized during compilation such that each intermediate representation is unique among the plurality of intermediate representations Each programmable logic controller is configured to receive an intermediate representation of the PLC program from the engineering system and compile the intermediate representation into PLC assembly code, wherein program structure of the intermediate representation is randomized during compilation with respect to usage of one or more program blocks.

Additional features and advantages of the invention will be made apparent from the following detailed description of illustrative embodiments that proceeds with reference to the accompanying drawings.

### BRIEF DESCRIPTION OF THE DRAWINGS

The foregoing and other aspects of the present invention are best understood from the following detailed description when read in connection with the accompanying drawings. For the purpose of illustrating the invention, there are shown in the drawings embodiments that are presently preferred, it being understood, however, that the invention is not limited to the specific instrumentalities disclosed. Included in the drawings are the following Figures:
FIG. 1 provides an example randomization done at an engineering system and the runtime system, according to some embodiments;
FIG. 2 provides an illustration of a Quad-Redundant Control Architecture, as it may be implemented in some embodiments;
FIG. 3 provides an illustration of PLC program randomization implemented through an API, according to some embodiments;
FIG. 4 illustrates an example randomization of legacy PLC projects, as it may be implemented in some embodiments;
FIG. 5 shows a data block randomization changes the address and offset of data block (DB) variables, which may be utilized in some embodiments;
FIG. 6 shows how DB block optimization eliminates memory padding and rearranges the memory layout, according to some embodiments;
FIG. 7 provides an illustration of how function block parameter reordering changes the binary representation of PLC programs, according to some embodiments;
FIG. 8 provides an illustration of cyclic variable reordering introduces binary changes to the PLC binary programs, according to some embodiments;
FIG. 9 illustrates a cyclic variable reordering introduces binary changes to the PLC binary programs, according to some embodiments; and
FIG. 10 provides an illustration of organization block randomization introduces variability in the timing and priority of OBs, according to some embodiments.

### DETAILED DESCRIPTION

Systems, methods, and apparatuses are described herein which relate generally to randomizing programs for cyber-attack resilient control in PLCs. More specifically, the techniques described herein allow for the execution of randomized binaries of PLC software in each of the instances of a redundant PLC architecture. This architecture is used to improve the system resilience (i.e., a factory and its subsystems) against failure modes originated by cyber-attacks. The architecture, referred to herein as REsilient CONtrol , or "RECON", relies on arrays of legacy PLCs as building blocks for creating a resilient Industrial Control Systems (ICS). Redundant PLCs are provided the same sensor inputs from the physical system and all PLCs execute a functionally identical program; however, each PLC contains a different randomized binary. In case of a successful exploit against the primary PLC, a fault is detected by the redundant system and a backup PLC takes control. Using different randomized binaries reduces the chances of the same exploit affecting the backup PLCs. Moreover, after a fault is detected, a new randomized binary can be re-compiled on-the-fly to the affected PLC instance to increase the availability of the system. As described in further detail below, in some embodiments of RECON, randomization occurs in two stages: (1) at the high-level compile time where PLC source code is transformed into the PLC intermediate representation; and (2) at runtime where the PLC intermediate representation is transformed into assembly instructions for execution in the PLC processor. The different randomization opportunities that each code representation offers may be leveraged to create a large combination of code and memory paths that enable a scalable RECON architecture.

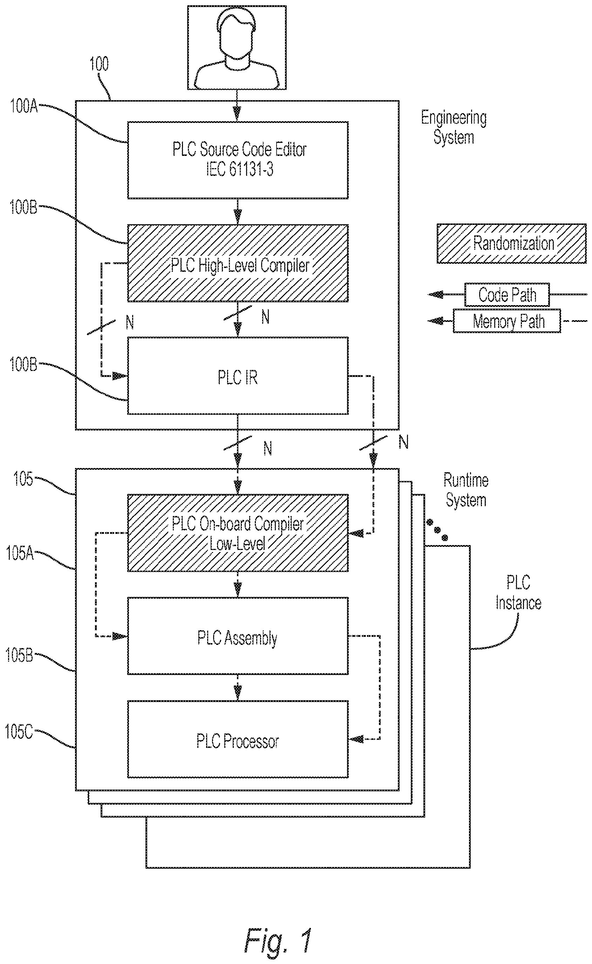

FIG. 1 provides a high-level overview of randomization performed at an Engineering System 100 (e.g., Siemens TIA Portal Engineering System) and a Runtime System 105 (e.g., Siemens PLC Operating System), according to some embodiments. PLCs are hard real-time control systems designed to interact with and control physical processes through sensors and actuators in harsh environments and for very long periods of time. PLCs are programmed in an Engineering System 100 using domain-specific, high-level PLC programming languages (e.g., IEC 61131-3) designed for physical system experts (i.e., electrical, mechanical engineers).

As shown in FIG. 1, the PLC source code is developed in a Source Code Editor 100A in the Engineering System 100. The source code compiled by a High-Level Compiler 100B into a PLC Intermediate Representation (IR) 100C. The PLC IR 100C allows the Engineering System 100 to target multiple PLC versions with different hardware and firmware. In some embodiments, multiple PLC languages are used to develop the program. An Integrated Development Environment (e.g., part of the Source Code Editor 100A) compiles the various source code languages into the PLC IR 100C that is retargetable to various PLC architectures (e.g., x86, MIPS, ARM, etc.). Abstract Syntax Tree (AST) randomization of the code and memory layout may additionally be performed.

To deploy the PLC program to the field, the PLC IR 100C is "downloaded" by the Engineering System 100 into the Runtime System 105 of the PLC; the Runtime System 105 is analogous to an operating system in a general purpose computer. The Runtime System then compiles the PLC IR into PLC Assembly code using an On-Board Compiler 105A (included in firmware of the PLC). Finally, the PLC Assembly 105B is executed by the PLC Processor 105C in a cyclic manner. Notice that in the case of a traditional resilient PLC architecture, the same PLC IR is distributed to all PLC instances, and assuming that all instances have the same version of the on-board compiler, all instances generate identical PLC Assembly for the common PLC IR.

To improve the fault-tolerance against faults caused by deliberate and pervasive cyber-attacks, in some embodiments RECON utilizes code and memory path randomization in the high-level and in the on-board compilers. High-level compiler randomization takes advantage of a suite of rich compiler transformation and optimizations done at the Abstract Syntax Tree (AST) and Control Flow Graph (CFG) levels. The first randomization step introduces high variability at the PLC IR level.

The second randomization step done in the on-board compiler has more restrictions imposed by the target assembly language and has less flexibility than the randomization done at a higher-level. However, the on-board compiler randomization provides a second line of defense to the system in case that an attacker compromises the first-stage of randomization. The on-board randomization mutates the PLC IR into a unique binary that cannot be accessed outside of the PLC. One way to further protect any outsiders from reading the on-board randomized binary is through PLC configuration. Thus, the on-board randomization hides the binary during the software development and maintenance cycle. Moreover, it should be noted that on-board randomization offers the ability to redeploy after failover using a newly randomized binary that is functionally equivalent to the prior version of the PLC software. In this way, a PLC that succumbs to cyberattack at the code level can re-launch with additional security measures which prevent the attack from being repeated.

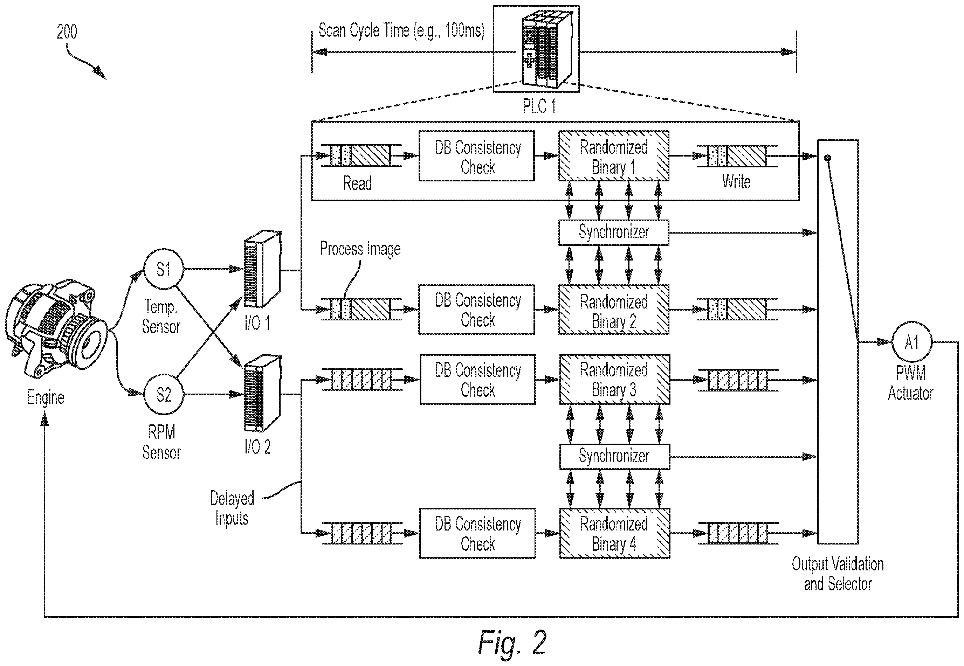

FIG. 2 shows a Quad-Redundant Control architecture 200, according to some embodiments. State-of-the-art PLCs provide more computation power than legacy PLCs with the use of multi-core processors. The Quad-Redundant Control PLC architecture 200 shown in FIG. 2 may be implemented, for example, using a plurality of dual-core PLCs (SIMATIC S7-1500). In addition of providing more logical PLCs, a benefit of this architecture is the virtualization of the PLCs in multi-core where each PLC has a disjointed memory and code spaces and prevents software failures in the master PLC to affect the slave PLC. Having virtualized dual-redundancy in the same core allows a faster recovery in case of a failure.

In FIG. 2, the Engine represents the physical system under control. Two sensors (S1, S2) measure the temperature and rotations-per-minute of the Engine. Two redundant I/O boards (I/O 1, I/O 2) connect the sensor data to the PLCs (PLC 1, ..., PLC 4). In every Scan Cycle Time (e.g., 100ms), the PLC executes three main stages: Read, Process, and Write. It is important to note that the Read and Write are not done directly to sensors and actuators, but through the PLC's Process Image. The Process Image is a buffer that decouples the execution from the physical process and allows the PLC to recover in case of any interrupts or faults. That is, the process image is guaranteed to be consistent in every cycle boundaries (e.g., 100ms, 200ms, 300ms). The Process Image is checked for consistency by the DB Consistency Check. In the Process stage, RECON uses different randomized binaries compiled using the method described in FIG. 1. Since the code and memory paths are different for every randomized binary, the Synchronizer checks for functional equivalence of the code executed in the master and slave PLCs. Functional equivalence may be accomplished through the insertion of runtime checkpoints at equivalent locations at various granularities. For example, if the randomization is done at the basic block granularity (e.g., micro-instruction shuffling), checkpoints can be inserted at basic block boundaries.

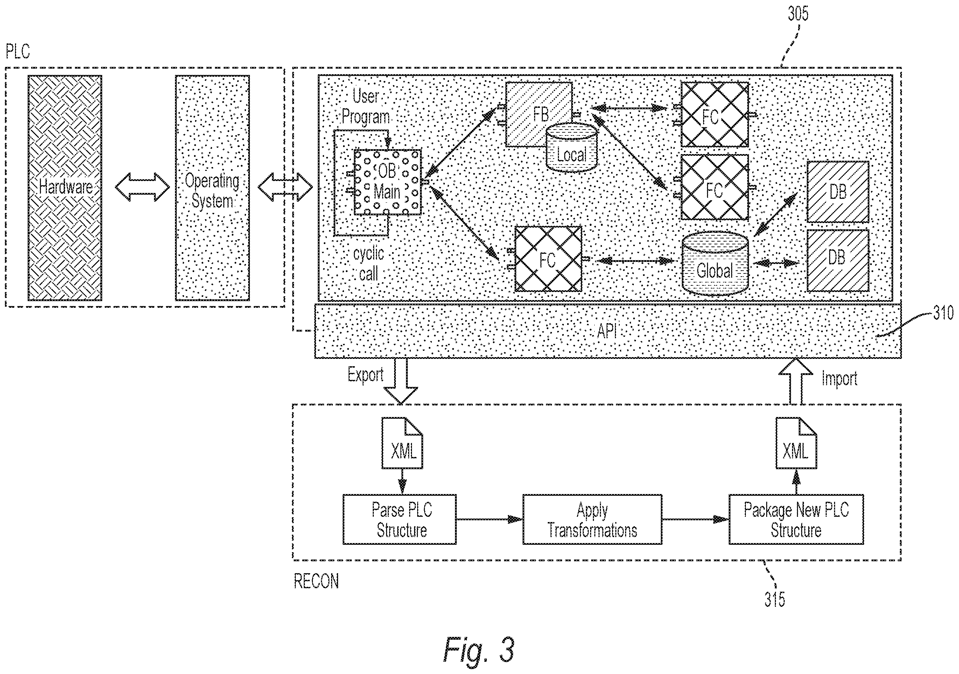

FIG. 3 is a system diagram which provides additional details of how PLC program randomization may be implemented, according to some embodiments. As is understood in the art, a PLC comprises an operating system and a user program. The operating system provides the intrinsic PLC functionality (e.g., handling of restart and errors, memory management, calling the user program, etc.). As shown in FIG. 3, the user program comprises all "blocks" that perform the automation task. The user program is programmed with program blocks including Function Blocks (FB) and Functions (FC). FBs and FCs reference local and global variables organized in Data Blocks (DB). In PLCs, the user program is typically executed cyclically (noncyclic programs are also possible) in an Organization Block (OB). The main cycle OB always exists and it is available when a PLC program is initialized and executed in an infinite loop.

The PLC program structure shown in FIG. 3 is fixed and provides a clear structure with different block types. This example illustrates the method to connect the RECON system to an Engineering System 305. RECON 315 in this example comprises software components which parse the PLC structure, apply transformations, and package a new PLC structure. More specifically, using an Application Programming Interface (API) 310, RECON 315 accesses and randomizes the PLC program before it is downloaded. Modifying the block structure of a PLC program has a profound effect in the generated code because the mappings of blocks (OB, FB, FC, DB) to binary representation change. Therefore, modification to the PLC program structure achieves the binary diversification sought by RECON. Using the general architecture set forth in FIG. 3, randomization of all block types in the PLC program structure may be achieved

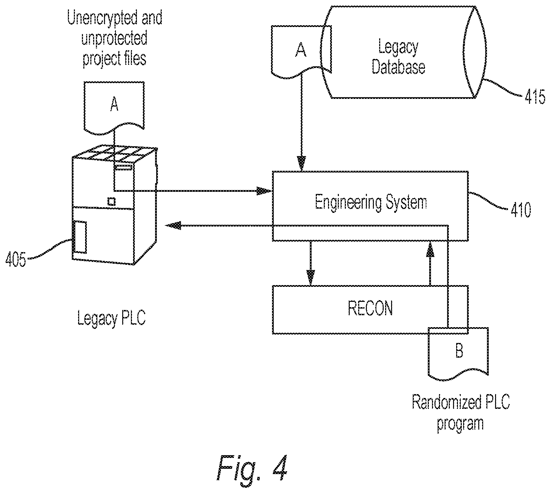

RECON may be used to perform PLC program randomization in both state-of-the-art and in legacy systems. FIG. 4 shows two techniques how randomization of legacy PLC projects may be performed using RECON, according to some embodiments. As shown in FIG. 4, the PLC programs can be extracted from a Legacy PLC 405 into the Engineering System 410. Alternatively, instead of extracting the PLC program from the Legacy PLC 405, a Legacy Database 415 can provide the PLC project files. The condition for either mechanism is that the project files are unencrypted and unprotected, as shown in FIG. 4.

Elaborating on the techniques described above, PLC program randomization is performed by three software components: a Data Block Randomization Component, a Function Block and Function Randomization Component, and an Organization Block Randomization Component. Each of these components is described in further detail below.

DBs organize the memory in addressable blocks that are used by the program to access variables, sensors, and actuators. A DB is a collection of named variables referred to as Tags. Tags are reserved memory areas for values in the PLC. Tags contain a data type (e.g., Bool, Integer, etc.), a value (e.g., "15"), and a physical address in the DB.

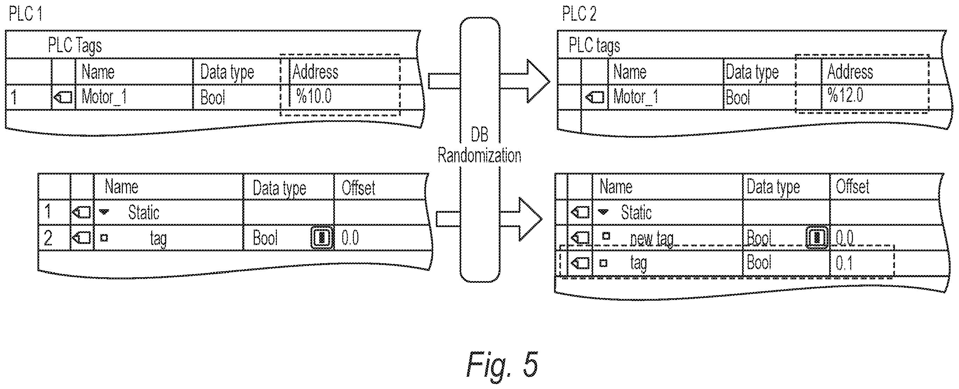

FIG. 5 illustrates how block randomization changes the address and offset of DB variables, according to some embodiments. As shown in FIG. 5, the "Motor_1" tag in PLC 1 has a Bool type and an address "%I0.0". After processing by the Data Block Randomization Component, the address of the same variable in PLC 2 can be changed to "%I2.0". Similarly, the offset in static variable "tag" in PLC 1 of Bool type can be changed from "0.0" to "0.1" by introducing the "new_tag" before "tag" in the symbol table in PLC2. These changes diversify the memory layout in a PLC in such a way that the same cyber-exploit is less likely to be effective in two PLCs with different memory layouts.

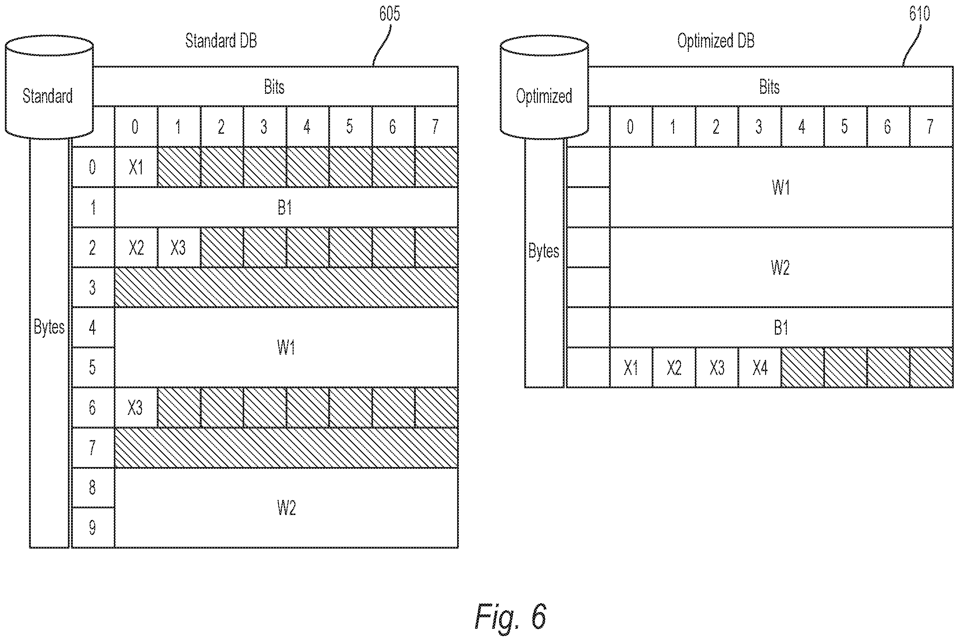

An additional layer of randomization is to enable the DB block optimization where all tags are sorted by their data type. The sorting minimizes the data gaps (padding) between tags and optimizes the memory access for the target PLC processor as shown in FIG. 6. In this example, the Standard DB 605 has data gaps between variables that are eliminated in the Optimized DB 610. In combination, the two presented DB randomization techniques allow RECON to generate hundreds of equivalent memory layouts for PLC diversified programs.

The Function Block and Function Randomization Component changes the structure of both the code and memory stack, and therefore it is important for RECON to create program variations. The difference between FB and FC lies in the cyclic data storage. FCs are blocks without data storage. This means that the values FCs are stateless and the block variables cannot be persisted over cycles. And FBs, on the other hand, are blocks with cyclic data storage. This means that FBs are stateful and block variable values are persisted over cycles. Both FB and FC are programmed using a variety of high-level languages including graphical and textual languages. The most popular PLC programming language is the Structured Control Language (SCL) which is based on PASCAL. SCL is also based on the Structured Text (ST) specification for PLC programming standardized by the IEC 61131-3. The FB and FC randomization techniques described below are based on SCL for illustration purposes; however, it should be understood that these techniques may be generalized and applied to any PLC programming language which provides similar functionality.

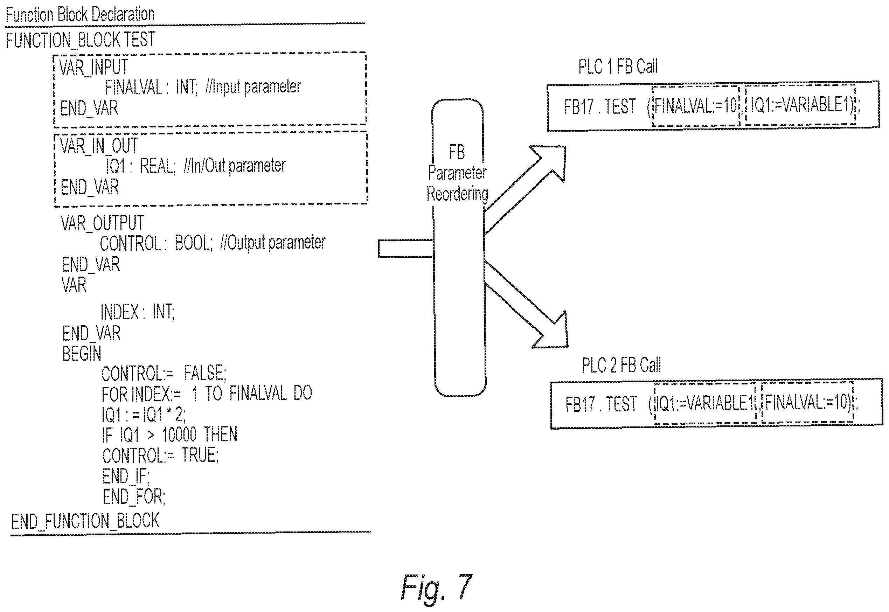

Function blocks and functions have a declaration comprising input, output, in-out parameters, and the body of the function. This declaration is common to all languages, including SCL. FIG. 7 shows an exemplary function block declaration for a "TEST" program comprising an input parameter "FINALVAL", an in-out parameter "IQ1", and an output parameter "CONTROL". One technique to introduce randomization is to reorder the parameters when calling the function block as shown by the two examples on the right. Because the parameters are named, the order in which the parameters are listed does not change the meaning of the program. However, this creates a different memory layout for the two programs. In real PLC programs, the number of parameters tends to be very large (sometimes hundreds of parameters), and therefore this technique can be very effective in creating a large number of different but equivalent SCL programs.

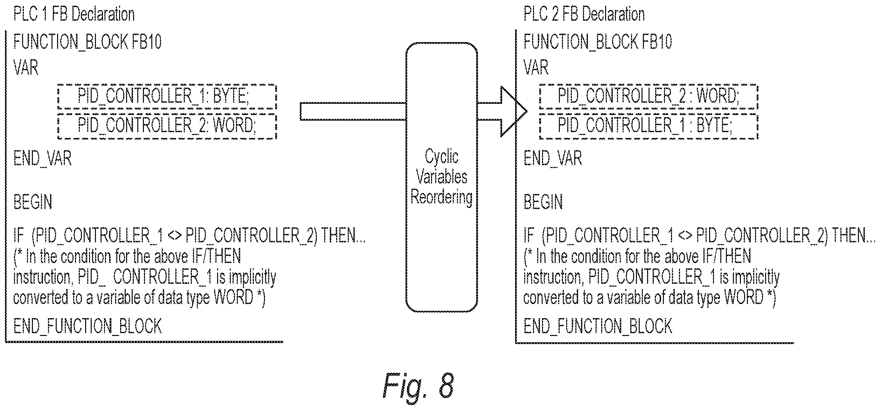

A similar technique is applicable to FB's cyclic variables that are persisted across cycles. FIG. 8 illustrates how cyclic variable reordering introduces binary changes to the PLC binary programs, according to some embodiments. As shown in FIG. 8, the order of the variables PID_CONTROLLER_1 and PID_CONTROLLER_2 can be rearranged such that cyclic variables occupy different memory locations in different PLC instances.

The SCL code itself can be randomized in both the control flow and data flow levels. For example, for the control flow, the extensible markup language (XML) file containing the SCL statements may be parsed and the control flow graph may be reconstructed where conditional statements are transformed into equivalent control flow constructs.

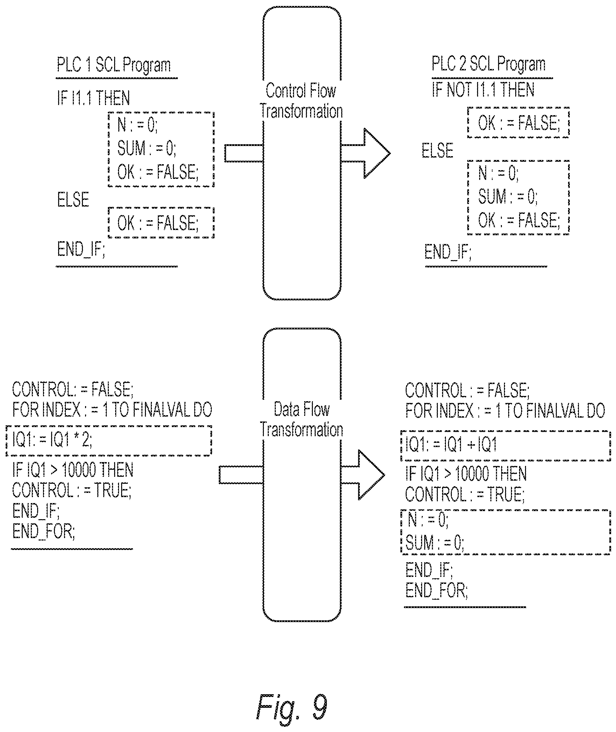

FIG. 9 shows how control flow and data flow transformations introduce binary diversification at the SCL code level, according to some embodiments. In FIG. 9, for example, the structure of the if-else statement is inverted with a "NOT" instruction such that the body of the "if' statement in PLC 1 becomes the body of the else statement in PLC 2, and vice-versa. For data flow, the XML file containing the SCL statement may be parsed, and the data flow graph may reconstructed where expressions are transformed into equivalent data flow expressions. For example, FIG. 7 shows that the expression "IQ1 := IQ1 ^{∗} 2" in PLC 1 is transformed into "IQ1 := IQ1 + IQ1" in PLC 2. Additionally, additional expressions such as "N := 0" and "SUM := 0" may be inserted that do not contribute to the control program but generate binary diversity at the code level when compiled. This is similar to a NOOP (no-operation) randomization technique. It is important to note that NOOP operands do not exist in some PLCs but inserting operations that do not contribute functionally to the program is functionally equivalent.

In some embodiments, an ordering randomization technique for FBs and FCs is applied that affects their arrangement in memory. Every FB and FC has a triple <name, type, number>, where name is an arbitrary name provided by the user in the PLC program, the type is an enumeration depending on the block type (e.g., FB, FC, OB, DB), and number is a unique identifier associated to the block. The key observation is that the locations of blocks in the PLC memory correspond to their number as they are first sorted and then downloaded to the PLC for execution. Thus, an ordering randomization technique may be applied to randomize the number in the triples to change their order during execution. Additionally (or alternatively), dummy FCs and FBs may be inserted to take up memory space and arbitrarily move other FBs and FCs that provide useful work. This transformation is also possible through the open interfaces and can also be extended to DBs.

OBs are the interface between the operating system and the user program. User programs can only be called by an OB and therefore are an important part of the PLC program structure. OBs are called by the operating system cyclically or when certain events occur. In most PLCs, there are several types of OBs (or similar concept in other vendors) that the user can chose for their programs depending on the desired functionality. For example, there are startup OBs, cyclic program execution OBs, interrupt (time-of-day, cyclic, time-delay, hardware) OBs, and error (asynchronous and synchronous) OBs. OBs are identified by a number, and different numbers have different priorities. For example, "OB 1" is the cyclic program OB with the highest priority, and OB 10 is a time-of-day interrupt OB with a lower priority. For example, Some PLCs provide multiple interrupts of the same type, for example, in Siemens PLCS, OB 32, OB 33, OB 34, and OB 35 are cyclic interrupt OBs that users have at their disposal to organize their program.

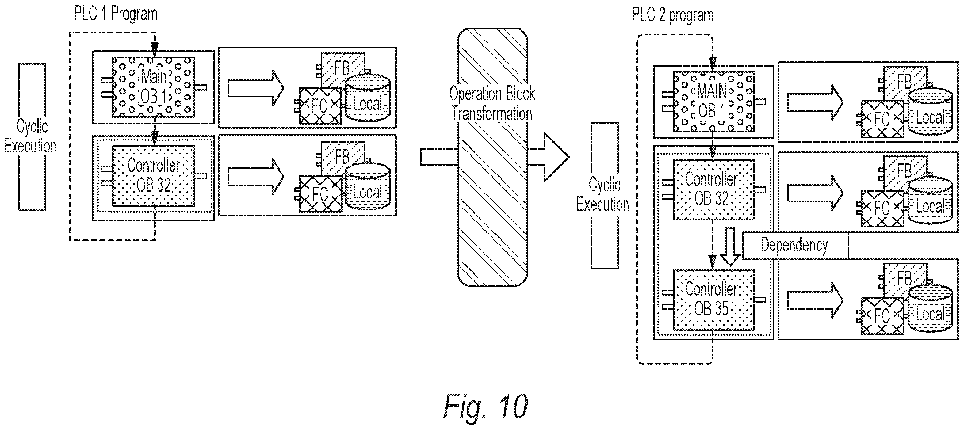

The Organization Block Randomization Component reorders the OBs such that different PLC programs use different OBs (with the same priority and real-time guarantees). For example, FIG. 10 shows how a PLC program structure with two OBs (OB 1 and OB 32) can be transformed into a program structure with three OBs (OB 1, OB 32, and OB 35). In this example, the Controller code is split into two and the first half is assigned to OB 32, and the second half is assigned to OB 35. Notice that this creates a dependency between OB 35 and OB 32. However, synchronization between OBs is supported in PLCs. FIG. 10 also illustrates that the randomization that RECON applies to OB, FB, FC, and DB to create a large combinatorial space can be leveraged to create diversified PLC binaries.

The processors described herein as used by control layer devices may include one or more central processing units (CPUs), graphical processing units (GPUs), or any other processor known in the art. More generally, a processor as used herein is a device for executing machine-readable instructions stored on a computer readable medium, for performing tasks and may comprise any one or combination of, hardware and firmware. A processor may also comprise memory storing machine-readable instructions executable for performing tasks. A processor acts upon information by manipulating, analyzing, modifying, converting or transmitting information for use by an executable procedure or an information device, and/or by routing the information to an output device. A processor may use or comprise the capabilities of a computer, controller or microprocessor, for example, and be conditioned using executable instructions to perform special purpose functions not performed by a general purpose computer. A processor may be coupled (electrically and/or as comprising executable components) with any other processor enabling interaction and/or communication there-between. A user interface processor or generator is a known element comprising electronic circuitry or software or a combination of both for generating display images or portions thereof. A user interface comprises one or more display images enabling user interaction with a processor or other device.

Various devices described herein including, without limitation, the control layer devices and related computing infrastructure, may include at least one computer readable medium or memory for holding instructions programmed according to embodiments of the invention and for containing data structures, tables, records, or other data described herein. The term "computer readable medium" as used herein refers to any medium that participates in providing instructions to one or more processors for execution. A computer readable medium may take many forms including, but not limited to, non-transitory, non-volatile media, volatile media, and transmission media. Non-limiting examples of non-volatile media include optical disks, solid state drives, magnetic disks, and magneto-optical disks. Non-limiting examples of volatile media include dynamic memory. Non-limiting examples of transmission media include coaxial cables, copper wire, and fiber optics, including the wires that make up a system bus. Transmission media may also take the form of acoustic or light waves, such as those generated during radio wave and infrared data communications.

An executable application, as used herein, comprises code or machine readable instructions for conditioning the processor to implement predetermined functions, such as those of an operating system, a context data acquisition system or other information processing system, for example, in response to user command or input. An executable procedure is a segment of code or machine readable instruction, sub-routine, or other distinct section of code or portion of an executable application for performing one or more particular processes. These processes may include receiving input data and/or parameters, performing operations on received input data and/or performing functions in response to received input parameters, and providing resulting output data and/or parameters.

The functions and process steps herein may be performed automatically, wholly or partially in response to user command. An activity (including a step) performed automatically is performed in response to one or more executable instructions or device operation without user direct initiation of the activity.

The system and processes of the figures are not exclusive. Other systems, processes and menus may be derived in accordance with the principles of the invention to accomplish the same objectives. Although this invention has been described with reference to particular embodiments, it is to be understood that the embodiments and variations shown and described herein are for illustration purposes only. As described herein, the various systems, subsystems, agents, managers and processes can be implemented using hardware components, software components, and/or combinations thereof.

## Claims

1. A method for programmable logic controller (PLC) program randomization in a redundant PLC architecture system, the method comprising:
a) receiving, by an engineering system (100, 305) computer, source code (100A) corresponding to a PLC program;
b) compiling, by the engineering system computer (105, 105A), the source code into a plurality of functionally equivalent intermediate representations (100C) of the PLC program, wherein the program structure of the PLC program is randomized during compilation utilizing code and memory path randomization such that each intermediate representation (100C) is unique among the plurality of intermediate representations; and
c) transmitting, by the engineering system computer, the plurality of intermediate representations to one or more PLCs (105C); and
d1) receiving, by the PLC, a first intermediate representation of the PLC program;
d2) compiling, by the PLC, the first intermediate representation into a PLC assembly code, wherein the program structure of the first intermediate representation is randomized during compilation; and
d3) executing, by the PLC, the PLC assembly code; and
e1) following failover of the PLC, re-compiling the first intermediate representation into a new PLC assembly code, wherein the program structure of the first intermediate representation is randomized during re-compilation; and
e2) executing, by the PLC, the new PLC assembly code; wherein
f) randomization occurs in two stages: (1) at the high-level compile time where the PLC source code is transformed into the PLC intermediate representation; and (2) at runtime where the PLC intermediate representation is transformed into assembly instructions for execution in the PLC processor.

2. The method of claim 1, wherein the program structure of the PLC program is randomized during compilation by randomizing a memory layout of a plurality of data blocks used by the PLC program.

3. The method of claim 2, wherein the memory layout of plurality of data blocks is randomized by assigning a unique memory address to each data block.

4. The method claim 3, further comprising:
sorting the plurality of data blocks by type during compilation to optimize memory access in the memory layout.

5. The method of claim 1, wherein the program structure of the PLC program is randomized during compilation by randomizing usage of a plurality of function blocks used by the PLC program.

6. The method of claim 4, wherein randomization of usage of the plurality of function blocks used by the PLC program comprises:
randomizing ordering of parameters used in calling each function block.

7. The method of claim 5, wherein randomization of usage of the plurality of function blocks used by the PLC program comprises:
randomizing ordering cyclic variables used by each function block.

8. The method of claim 5, wherein randomization of usage of the plurality of function blocks used by the PLC program comprises randomizing control flow of each function block by:
constructing a control flow graph where conditional statements are transformed into equivalent control flow constructs; and
randomizing ordering of the control flow constructs.

9. The method of claim 5, wherein randomization of usage of the plurality of function blocks used by the PLC program comprises randomizing data flow of each function block by:
constructing a data flow graph where conditional statements are transformed into equivalent data flow expressions; and
randomizing ordering of the data flow expressions.

10. The method of claim 5, wherein randomization of usage of the plurality of function blocks used by the PLC program comprises inserting one or more non-functional function blocks into the plurality of function blocks.

11. The method of claim 1, wherein the PLC program comprises a plurality of function blocks, each identified by a triple comprising a numerical identifier and the program structure of the PLC program is randomized by:
assigning a random value to each numerical identifier, and
sorting the plurality of function blocks by numerical identifier.

12. The method of claim 1, wherein program structure of the PLC program is randomized during compilation by reordering one or more organization blocks in the PLC program.

13. A system for programmable logic controller (PLC) program randomization in a redundant PLC architecture system, the system comprising:
a computer readable storage medium storing source code corresponding to a PLC program;
an engineering system (100) configured to compile the source code into a plurality of functionally equivalent intermediate representations of the PLC program, wherein program structure of the PLC program is randomized during compilation utilizing code and memory path randomization such that each intermediate representation is unique among the plurality of intermediate representations; and
a plurality of programmable logic controllers, each programmable logic controller configured to:
receive an intermediate representation of the PLC program from the engineering system
compile the intermediate representation into PLC assembly code, wherein program structure of the intermediate representation is randomized during compilation with respect to usage of one or more program blocks;
receive, by the PLC, a first intermediate representation of the PLC program;
compile, by the PLC, the first intermediate representation into a PLC assembly code, wherein the program structure of the first intermediate representation is randomized during compilation; and
execute, by the PLC, the PLC assembly code; and
following failover of the PLC, re-compile the first intermediate representation into a new PLC assembly code, wherein the program structure of the first intermediate representation is randomized during re-compilation; and
executing, by the PLC, the new PLC assembly code; wherein
randomization occurs in two stages: (1) at the high-level compile time where the PLC source code is transformed into the PLC intermediate representation; and (2) at runtime where the PLC intermediate representation is transformed into assembly instructions for execution in the PLC processor.

## Patentansprüche

1. Verfahren für eine Programmrandomisierung durch speicherprogrammierbare Steuerung (PLC-Programmrandomisierung) in einem redundanten PLC-Architektursystem, wobei das Verfahren Folgendes umfasst:
a) Empfangen durch einen Engineering-Systemcomputer (100, 305) von Quellcode (100A), der einem PLC-Programm entspricht;
b) Kompilieren durch den Engineering-Systemcomputer (105, 105A) des Quellcodes in mehrere funktionell äquivalente Zwischenrepräsentationen (100C) des PLC-Programms, wobei die Programmstruktur des PLC-Programms während der Kompilierung unter Verwendung von Code und einer Speicherwegrandomisierung derart randomisiert wird, dass jede Zwischenrepräsentation (100C) unter den mehreren Zwischenrepräsentationen einzigartig ist; und
c) Senden durch den Engineering-Systemcomputer der mehreren Zwischenrepräsentationen an eine oder mehrere PLCs (105C); und
d1) Empfangen durch die PLC einer ersten Zwischenrepräsentation des PLC-Programms;
d2) Kompilieren durch die PLC der ersten Zwischenrepräsentation in einen PLC-Assembler-Code, wobei die Programmstruktur der ersten Zwischenrepräsentation während der Kompilierung randomisiert wird; und
d3) Ausführen durch die PLC des PLC-Assembler-Codes; und
e1) nach Ausfallsicherung der PLC Neukompilieren der ersten Zwischenrepräsentation in einen neuen PLC-Assembler-Code, wobei die Programmstruktur der ersten Zwischenrepräsentation während der Neukompilierung randomisiert wird; und
e2) Ausführen durch die PLC des neuen PLC-Assembler-Codes; wobei
f) die Randomisierung in zwei Stufen erfolgt: (1) bei einer Kompilierzeit hoher Stufe, wo der PLC-Quellcode in die PLC-Zwischenrepräsentation umgewandelt wird; und (2) bei einer Laufzeit, wo die PLC-Zwischenrepräsentation in Assembler-Anweisungen für eine Ausführung in dem PLC-Prozessor umgewandelt wird.

2. Verfahren nach Anspruch 1, wobei die Programmstruktur des PLC-Programms während der Kompilierung durch Randomisierung eines Speicher-Layouts mehrerer Datenblöcke, die durch das PLC-Programm verwendet werden, randomisiert wird.

3. Verfahren nach Anspruch 2, wobei das Speicher-Layout mehrerer Datenblöcke durch Zuweisen einer einzigartigen Speicheradresse zu jedem Datenblock randomisiert wird.

4. Verfahren nach Anspruch 3, das ferner Folgendes umfasst:
Sortieren der mehreren Datenblöcke während der Kompilierung nach dem Typ, um einen Speicherzugriff in dem Speicher-Layout zu optimieren.

5. Verfahren nach Anspruch 1, wobei die Programmstruktur des PLC-Programms während der Kompilierung durch Randomisierung der Verwendung mehrerer Funktionsblöcke, die durch das PLC-Programm verwendet werden, randomisiert wird.

6. Verfahren nach Anspruch 4, wobei die Randomisierung der Verwendung der mehreren Funktionsblöcke, die durch das PLC-Programm verwendet werden, Folgendes umfasst:
Randomisieren der Anordnung von Parametern, die bei Aufrufen jedes Funktionsblocks verwendet werden.

7. Verfahren nach Anspruch 5, wobei das Randomisieren der Verwendung der mehreren Funktionsblöcke, die durch das PLC-Programm verwendet werden, Folgendes umfasst:
Randomisieren der Anordnung von zyklischen Variablen, die durch jeden Funktionsblock verwendet werden.

8. Verfahren nach Anspruch 5, wobei die Randomisierung der Verwendung der mehreren Funktionsblöcke, die durch das PLC-Programm verwendet werden, umfasst, einen Steuerfluss jedes Funktionsblocks durch Folgendes zu randomisieren:
Erstellen eines Steuerflussgraphen, wo konditionale Aussagen in äquivalente Steuerflusskonstruktionen umgewandelt werden; und Randomisieren der Anordnung der Steuerflusskonstruktionen.

9. Verfahren nach Anspruch 5, wobei die Randomisierung der Verwendung der mehreren Funktionsblöcke, die durch das PLC-Programm verwendet werden, umfasst, einen Datenfluss jedes Funktionsblocks durch Folgendes zu randomisieren:
Erstellen eines Datenflussgraphen, wo konditionale Aussagen in äquivalente Datenflussausdrücke umgewandelt werden; und
Randomisieren der Anordnung der Datenflussausdrücke.

10. Verfahren nach Anspruch 5, wobei das Randomisieren der Verwendung der mehreren Funktionsblöcke, die durch das PLC-Programm verwendet werden, umfasst, einen oder mehrere nicht funktionelle Blöcke in die mehreren Funktionsblöcke einzufügen.

11. Verfahren nach Anspruch 1, wobei das PLC-Programm mehrere Funktionsblöcke umfasst, die jeweils durch eine Dreiergruppe identifiziert werden, die eine numerische Kennung umfasst, und die Programmstruktur des PLC-Programms durch Folgendes randomisiert wird:
Zuweisen eines Zufallswerts zu jeder numerischen Kennung, und Sortieren der mehreren Funktionsblöcke durch eine numerische Kennung.

12. Verfahren nach Anspruch 1, wobei die Programmstruktur des PLC-Programms während der Kompilation durch Neuordnen eines oder mehrerer Organisationsblöcke in dem PLC-Programm randomisiert wird.

13. System für eine Programmrandomisierung durch speicherprogrammierbare Steuerung (PLC-Programmrandomisierung) in einem redundanten PLC-Architektursystem, wobei das System Folgendes umfasst:
ein computerlesbares Speichermedium, das einen Quellcode speichert, der einem PLC-Programm entspricht;
ein Engineering-System (100), das konfiguriert ist, den Quellcode in mehrere funktionell äquivalente Zwischenrepräsentationen des PLC-Programms zu kompilieren, wobei die Programmstruktur des PLC-Programms während der Kompilierung unter Verwendung von Code und einer Speicherwegrandomisierung derart randomisiert wird, dass jede Zwischenrepräsentation unter den mehreren Zwischenrepräsentationen einzigartig ist; und
mehrere speicherprogrammierbare Steuereinheiten, wobei jede speicherprogrammierbare Steuereinheit konfiguriert ist zum:
Empfangen einer Zwischenrepräsentation des PLC-Programms von dem Engineering-System;
Kompilieren der Zwischenrepräsentation in einen PLC-Assembler-Code, wobei die Programmstruktur der ersten Zwischenrepräsentation während der Kompilierung in Bezug auf die Verwendung eines oder mehrerer Programmblöcke randomisiert wird;
Empfangen durch die PLC einer ersten Zwischenrepräsentation des PLC-Programms;
Kompilieren durch die PLC der ersten Zwischenrepräsentation in einen PLC-Assembler-Code, wobei die Programmstruktur der ersten Zwischenrepräsentation während der Kompilierung randomisiert wird; und
Ausführen durch die PLC des PLC-Assembler-Codes; und
nach Ausfallsicherung der PLC Neukompilieren der ersten Zwischenrepräsentation in einen neuen PLC-Assembler-Code, wobei die Programmstruktur der ersten Zwischenrepräsentation während der Neukompilierung randomisiert wird; und
Ausführen durch die PLC des neuen PLC-Assembler-Codes; wobei die Randomisierung in zwei Stufen erfolgt: (1) bei einer Kompilierzeit hoher Stufe, wo der PLC-Quellcode in die PLC-Zwischenrepräsentation umgewandelt wird; und (2) bei einer Laufzeit, wo die PLC-Zwischenrepräsentation in Assembler-Anweisungen für eine Ausführung in dem PLC-Prozessor umgewandelt wird.

## Revendications

1. Procédé de randomisation de programme d'automate programmable industriel (API) dans un système à architecture d'API redondants, le procédé comprenant :
a) la réception, par un ordinateur de système d'ingénierie (100, 305), d'un code source (100A) correspondant à un programme d'API ;
b) la compilation, par l'ordinateur de système d'ingénierie (105, 105A), du code source en une pluralité de représentations intermédiaires fonctionnellement équivalentes (100C) du programme d'API, la structure de programme du programme d'API étant randomisée durant la compilation par randomisation de code et de trajet de mémoire de telle sorte que chaque représentation (100C) soit unique parmi la pluralité de représentations intermédiaires ; et
c) la transmission, par l'ordinateur de système d'ingénierie, de la pluralité de représentations intermédiaires à un ou plusieurs API (105C) ; et
d1) la réception, par l'API, d'une première représentation intermédiaire du programme d'API ;
d2) la compilation, par l'API, de la première représentation intermédiaire en un code d'assemblage d'API, la structure de programme de la première représentation intermédiaire étant randomisée durant la compilation ; et
d3) l'exécution, par l'API, du code d'assemblage d'API ; et
e1) suite à une défaillance de l'API, la recompilation de la première représentation intermédiaire en un nouveau code d'assemblage d'API, la structure de programme de la première représentation intermédiaire étant randomisée durant la recompilation ; et
e2) l'exécution, par l'API, du nouveau code d'assemblage d'API ; dans lequel
f) la randomisation se déroule en deux étapes : (1) au moment de la compilation de haut niveau où le code source d'API est transformé en la représentation intermédiaire d'API ; et (2) au moment de l'exécution où la représentation intermédiaire d'API est transformée en instructions d'assemblage pour l'exécution dans le processeur d'API.

2. Procédé selon la revendication 1, dans lequel la structure de programme du programme d'API est randomisée durant la compilation par randomisation d'un agencement mémoire d'une pluralité de blocs de données utilisée par le programme d'API.

3. Procédé selon la revendication 2, dans lequel l'agencement mémoire de la pluralité de blocs de données est randomisé en attribuant une adresse mémoire unique à chaque bloc de données.

4. Procédé selon la revendication 3, comprenant en outre :
le classement par type de la pluralité de blocs de données durant la compilation pour optimiser l'accès mémoire dans l'agencement mémoire.

5. Procédé selon la revendication 1, dans lequel la structure de programme du programme d'API est randomisée durant la compilation par randomisation de l'utilisation d'une pluralité de blocs de fonctions utilisée par le programme d'API.

6. Procédé selon la revendication 4, dans lequel la randomisation de l'utilisation de la pluralité de blocs de fonctions utilisée par le programme d'API comprend :
la randomisation de l'ordre de paramètres utilisés dans l'appel de chaque bloc de fonction.

7. Procédé selon la revendication 5, dans lequel la randomisation de l'utilisation de la pluralité de blocs de fonctions utilisée par le programme d'API comprend :
la randomisation de l'ordre de variables cycliques utilisées par chaque bloc de fonction.

8. Procédé selon la revendication 5, dans lequel la randomisation de l'utilisation de la pluralité de blocs de fonctions utilisée par le programme d'API comprend la randomisation du flux de commande de chaque bloc de fonction en
construisant un graphe de flux de commande où des déclarations conditionnelles sont transformées en constructions de flux de commande équivalentes ; et
randomisant l'ordre des constructions de flux de commande.

9. Procédé selon la revendication 5, dans lequel la randomisation de l'utilisation de la pluralité de blocs de fonctions utilisée par le programme d'API comprend la randomisation du flux de données de chaque bloc de fonction en :
construisant un graphe de flux de données où des déclarations conditionnelles sont transformées en expressions de flux de données équivalentes ; et
randomisant l'ordre des expressions de flux de données.

10. Procédé selon la revendication 5, dans lequel la randomisation de l'utilisation de la pluralité de blocs de fonctions utilisée par le programme d'API comprend l'insertion d'un ou plusieurs blocs de fonction non fonctionnels dans la pluralité de blocs de fonction.

11. Procédé selon la revendication 1, dans lequel le programme d'API comprend une pluralité de blocs de fonction, identifiés chacun par un triplet comprenant un identifiant numérique et la structure de programme du programme d'API est randomisée en :
attribuant une valeur aléatoire à chaque identifiant numérique, et
classant la pluralité de blocs de fonction par identifiant numérique.

12. Procédé selon la revendication 1, dans lequel la structure de programme du programme d'API est randomisée durant la compilation en remettant en ordre un ou plusieurs blocs d'organisation dans le programme d'API.

13. Système de randomisation de programme d'automate programmable industriel (API) dans un système à architecture d'API redondants, le système comprenant :
un support de mémorisation lisible par ordinateur mémorisant le code source correspondant à un programme d'API ;
un système d'ingénierie (100) configuré pour compiler le code source en une pluralité de représentations intermédiaires fonctionnellement équivalentes du programme d'API, dans lequel la structure de programme du programme d'API est randomisée durant la compilation par randomisation de code et de trajet de telle sorte que chaque représentation intermédiaire soit unique parmi la pluralité de représentations intermédiaires ; et
une pluralité d'automates programmables industriels, chaque automate programmable industriel étant configuré pour :
recevoir une représentation intermédiaire du programme d'API à partir du système d'ingénierie ;
compiler la représentation intermédiaire en un code d'assemblage d'API, la structure de programme de la représentation intermédiaire étant randomisée durant la compilation relativement à l'utilisation d'un ou plusieurs blocs de programme ;
recevoir, par l'API, une première représentation intermédiaire du programme d'API ;
compiler, par l'API, la première représentation intermédiaire en un code d'assemblage d'API, la structure de programme de la première représentation intermédiaire étant randomisée durant la compilation ; et
exécuter, par l'API, le code d'assemblage d'API ; et
après une défaillance de l'API, recompiler la première représentation intermédiaire en un nouveau code d'assemblage d'API, la structure de programme de la première représentation intermédiaire étant randomisée durant la recompilation ; et
exécuter, par l'API, le nouveau code d'assemblage d'API ; dans lequel
la randomisation se déroule en deux étapes : (1) au moment de la compilation de haut niveau où le code source d'API est transformé en la représentation intermédiaire d'API ; et (2) au moment de l'exécution où la représentation intermédiaire d'API est transformée en instructions d'assemblage pour l'exécution dans le processeur d'API.
